# EUROPEAN PATENT SPECIFICATION

(11) **EP 3 363 355 B1**
(45) Date of publication and mention of the grant of the patent: **07.05.2025**
(21) Application number: 18156787.6
(22) Date of filing: 14.02.2018
(51) Int. Cl.: A61B 5/00, A61M 25/00

(54) **ELECTROPHYSIOLOGIC DEVICE AND METHOD FOR CONSTRUCTION THE ELECTROPHYSIOLOGIC DEVICE**
ELEKTROPHYSIOLOGISCHE VORRICHTUNG UND VERFAHREN ZUR KONSTRUKTION DER ELEKTROPHYSIOLOGISCHEN VORRICHTUNG
DISPOSITIF ÉLECTROPHYSIOLOGIQUE ET PROCÉDÉ DE CONSTRUCTION DU DISPOSITIF ÉLECTROPHYSIOLOGIQUE

(30) Priority: 15.02.2017 US 201715433357
(43) Date of publication of application: 22.08.2018
(73) Proprietor: Biosense Webster (Israel) Ltd., Yokneam 2066717 (IL)
(72) Inventor: TANG, Raymond Yue-Sing, Irwindale, CA California 91706 (US); HARO, Anabel, Irwindale, CA California 91706 (US)
(74) Representative: Carpmaels & Ransford LLP

(56) References cited:
- EP-A1- 1 530 981
- EP-A1- 3 184 044
- EP-A2- 2 471 455
- WO-A1-2016/149388
- WO-A2-2004/045698
- US-A1- 2002 095 202
- US-A1- 2003 217 463
- US-A1- 2014 052 120
- US-B1- 6 758 830

## Description

### FIELD OF THE PRESENT DISCLOSURE

This invention relates to sensors for use with electrophysiologic (EP) catheters, guiding sheaths and related devices for mapping and/or ablation of locations within a patient, such as the heart, in particular, to construction techniques for forming components including electrodes and navigation coils.

### BACKGROUND

Mapping of electrical potentials in the heart is now commonly performed, using cardiac catheters comprising electrophysiological sensors for mapping the electrical activity of the heart. Typically, time-varying electrical potentials in the endocardium are sensed and recorded as a function of position inside the heart, and then used to map a local electrogram or local activation time. Activation time differs from point to point in the endocardium due to the time required for conduction of electrical impulses through the heart muscle. The direction of this electrical conduction at any point in the heart is conventionally represented by an activation vector, which is normal to an isoelectric activation front, both of which may be derived from a map of activation time. The rate of propagation of the activation front through any point in the endocardium may be represented as a velocity vector. Mapping the activation front and conduction fields aids the physician in identifying and diagnosing abnormalities, such as ventricular and atrial tachycardia and ventricular and atrial fibrillation, which may result from areas of impaired electrical propagation in the heart tissue.

Localized defects in the heart's conduction of activation signals may be identified by observing phenomena such as multiple activation fronts, abnormal concentrations of activation vectors, or changes in the velocity vector or deviation of the vector from normal values. Examples of such defects include re-entrant areas, which may be associated with signal patterns known as complex fractionated electrograms. Once a defect is located by such mapping, it may be ablated (if it is functioning abnormally) or otherwise treated so as to restore the normal function of the heart insofar as is possible. As an illustration, cardiac arrhythmias including atrial fibrillation, may occur when regions of cardiac tissue abnormally conduct electric signals to adjacent tissue, thereby disrupting the normal cardiac cycle and causing asynchronous rhythm. Procedures for treating arrhythmia include disrupting the origin of the signals causing the arrhythmia, as well as disrupting the conducting pathway for such signals, such as by forming lesions to isolate the aberrant portion. Thus, by selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

Accordingly, a suitable EP catheter may have one or more sensor electrodes for measuring electrical signals and/or delivering ablation energy. Each electrode requires its own lead to conduct the received electrical signals through the catheter for recording and processing by instrumentation coupled to the catheter or to transmit the energy for ablation. Further, the EP catheter may also employ one or more location sensors to help track the position of the catheter and determine placement of the electrodes within the patient. Thus, a number of leads may also be required for the location sensors. The multiplicity of leads are typically routed through a lumen in the polymeric tube forming the catheter and must be connected to their respective electrode or location sensor, which represents a significant investment of time and labor as well as being subject to a high failure rate. For example, each lead is fished out through an opening made in the catheter wall, stripped of insulation and welded or soldered to the component and the process is repeated. Moreover, the typical materials used for electrodes, such as platinum or iridium, contribute significantly to the overall cost of the catheter.

Accordingly, it would be desirable to provide a sensor construction that reduces or avoids the use of dedicated electrode materials. Similarly, it would be desirable to provide a more reliable connection between the leads and the components. Further, it would be desirable to deploy such components on a variety of electrophysiologic devices, including catheters, guiding sheaths and others. The techniques of this disclosure as described in the following materials satisfy these and other needs.

US2003/217463A1 describes a method and apparatus for manufacturing implantable electrodes having a controlled surface area and integral conductors. WO2016/149388A1 describes field concentrating antennas for magnetic position sensors. US6758830B1 describes a catheter positioning system. US2002/095202A1 describes a cardiac electrode catheter and a method of manufacturing the same. US2014/052120A1 describes an electrophysiology catheter design. WO2004/045698A2 describes a multilumen body for an implantable medical device. EP3184044A1 (cited under Article 54(3) EPC) and EP2471455A2 both describe a catheter with improved position and/or location sensing with the use of single axis sensors that are mounted directly along a length or portion of the catheter whose position/location is of interest. The magnetic based, single axis sensors are provided on a single axis sensor (SAS) assembly, which can be linear or nonlinear as needed. A catheter thus includes a catheter body and a distal member of a particular 2D or 3D configuration that is provided by a support member on which at least one, if not at least three single axis sensors, are mounted serially along a length of the support member. The magnetic-based sensor assembly including at least one coil member that is wrapped on the support member, wherein the coil member is connected via a joint region to a respective cable member adapted to transmit a signal providing location information from the coil member to a mapping and localization system. The joint region advantageously provides strain relief adaptations to the at least one coil member and the respective cable member from detaching. In EP3184044A1, the wire distal and proximal portions of each coil may extend proximally through a lumen of the catheter shaft. A through-hole is formed into the lumen through the sidewall of the catheter shaft portion (through a first layer, a braided mesh and a second layer) for each wire portion. As such, sleeves are not needed. The extruded first layer may be formed as a multi-lumened tubing with lumens (with use of one or more suitable mandrels). The through-hole may be formed to communicate with the lumen, such that the wire portions all pass through the dedicated lumen along the length of the catheter shaft.

### SUMMARY

The invention is as defined in the independent claims.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
FIG. 1 is a top plan view of an electrophysiologic catheter within a deflectable guiding sheath, according to one embodiment.
FIG. 2 is a detail view of a distal portion of a lead wire extending from an elongated body, according to one embodiment.
FIG. 3 is a cross sectional view of the elongated body shown in FIG. 2, according to one embodiment.
FIG. 4 is a schematic view of the distal portion of the lead wire helically wrapped around the elongated body, according to one embodiment.
FIG. 5 is a schematic view of a coil formed by tightening the helical wraps of lead wire, according to one embodiment.
FIG. 6 is a schematic view of a deflectable guiding sheath including a coil and electrophysiologic catheter with an electrode assembly positioned in the ostium of a pulmonary vein, according to one embodiment.
FIG. 7 is a schematic illustration of an invasive medical procedure using an electrophysiologic device with an elongated body having at least one coil, according to one embodiment.

### DETAILED DESCRIPTION

At the outset, it is to be understood that this disclosure is not limited to particularly exemplified materials, architectures, routines, methods or structures as such may vary. Thus, although a number of such options, similar or equivalent to those described herein, can be used in the practice or embodiments of this disclosure, the preferred materials and methods are described herein.

It is also to be understood that the terminology used herein is for the purpose of describing particular embodiments of this disclosure only and is not intended to be limiting.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details. In some instances, well known structures and devices are shown in block diagram form in order to avoid obscuring the novelty of the exemplary embodiments presented herein.

For purposes of convenience and clarity only, directional terms, such as top, bottom, left, right, up, down, over, above, below, beneath, rear, back, and front, may be used with respect to the accompanying drawings. These and similar directional terms should not be construed to limit the scope of the disclosure in any manner.

Unless defined otherwise, all technical and scientific terms used herein have the same meaning as commonly understood by one having ordinary skill in the art to which the disclosure pertains.

As noted above, certain types of electrical activity within a heart chamber are not cyclical. Examples include arterial flutter or arterial fibrillation, and ventricular tachycardia originating in scars in the wall of the ventricle that have resulted from infarcts. Such electrical activity is random from beat to beat. To analyze or 'map' this type of electrical activity, it is desirable to provide an electrophysiologic catheter with one or more electrodes to measure the electrical signals. Further, RF energy may be delivered to selected treatment areas for ablation based therapies, including for example, isolation of a source of irregular electrical signals by blocking electrical conduction. Correspondingly, one or more electrodes may be used to deliver ablation energy. Still further, the catheter may employ one or more location sensors to help visualize or otherwise determine the relative position of the catheter within the patient. According to the techniques of this disclosure, one or more of the electrodes and/or location sensors may be formed from a coil of wire wound around the elongated body of the electrophysiologic catheter. In some embodiments, the electrophysiologic catheter may be advanced through a guiding sheath to facilitate positioning the electrodes at a desired position within the patient. Notably, the positioning of the electrophysiologic catheter within a guiding sheath may block one or more electrodes or location sensor of the catheter depending on the relative longitudinal positioning of the catheter within the guiding sheath, hindering visualization or otherwise reducing the effectiveness of recording or delivering electrical signals. As such, one or more of the electrodes and/or location sensors may also be formed from a coil of wire wound around the elongated body of the guiding sheath. Deploying electrodes and/or location sensors on the guiding sheath permits their use regardless of the relative position of the catheter.

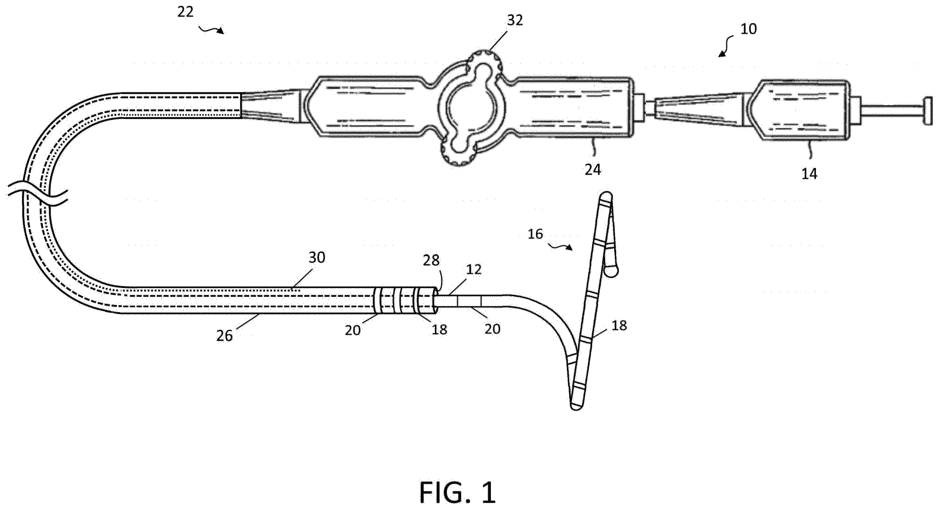

To provide a context for the disclosure, an exemplary catheter having a lasso-shaped electrode assembly coaxially disposed within a guiding sheath is shown schematically in FIG. 1. As will be appreciated, the techniques of this disclosure may be applied to any other catheter configurations having one or more electrodes and/or location sensors. Electrophysiologic catheter 10 comprises an elongated body 12 having proximal and distal ends, with control handle 14 at the proximal end of the catheter body and lasso electrode assembly 16 at the distal end. Lasso electrode assembly 16 may form a generally known or range-restricted angle that is substantially transverse to the longitudinal axis of elongated body 12.

Lasso electrode assembly 16 may be of a known fixed length, and comprises material that preferably is twistable but not stretchable when subjected to typical forces. In one aspect, lasso electrode assembly 16 may be sufficiently resilient so as to assume a predetermined, curved form, when no force is applied thereto, and to be deflected from the predetermined curved form when a force is applied thereto. For example, lasso electrode assembly 16 may have an elasticity that is generally constant over at least a portion of its length, for example, because of internal reinforcement of the curved section with a resilient longitudinal member, such as a shape memory material (e.g. a nickel-titanium alloy) as is known in the art. Lasso electrode assembly 16 may form a complete or partial lasso, i.e., as a preformed arcuate structure, which typically subtends between 180° and 360°. In one aspect, lasso electrode assembly 16 may form a substantially complete circle so as to allow mapping and/or ablation around or substantially around the circumference of a vessel. The radius of curvature of lasso electrode assembly 16, when unconstrained, may be typically between 7.5 mm and 15 mm. Because the arc structure is resilient and, possibly, slightly helical, when lasso electrode assembly 16 is positioned in the heart (against the ostium of pulmonary vein 50, for example),it will press against the heart tissue over the entire length of the arc, thus facilitating good tissue contact.

Lasso electrode assembly 16 may have one or more electrodes 18, configured as ring electrodes, and one or more location sensors 20. Conventionally, ring electrodes are rings formed from a suitable material, such as platinum or iridium, and positioned at various intervals along the length of a distal portion of elongated body 12. The ring electrodes are electrically connected, via electrode lead wires which extend through a lumen in the catheter, to electrical instruments, e.g., a monitor, stimulator or source of energy, e.g., RF energy, for ablation. Such conventional ring electrodes are connected to their lead wire by drawing the wire out of a lumen through an exit hole that extends from the lumen to the side surface of the catheter body. As noted above, the distal end of the electrode lead wire may be stripped of any nonconductive coating or insulation and then welded or soldered onto the inner surface of a ring electrode. The ring electrode is then slipped over the tip shaft to a position directly over the exit hole while drawing the electrode lead wire back into the lumen. The ring electrode is then secured in place, e.g., by swaging or by the application of an appropriate adhesive. A resin, e.g., polyurethane resin, is often applied to the margins or edges of the ring electrode to assure a smooth transition between the outer circumferential surface of the ring electrode and the outer circumferential surface of the catheter shaft. In contrast, one or more of electrodes 18 and location sensors 20 may be formed from a continuous length of wire extending from a proximal end and helically wrapped to form a coil around a distal portion of elongated body 12 as described in further detail below.

Further, FIG. 1 depicts electrophysiologic catheter 10 advanced coaxially within a deflectable guiding sheath 22, having a control handle 24 and an elongated body 26 with at least one inner lumen 28 sized to accommodate elongated body 12 of catheter 10. Guiding sheath 22 may also have one or more electrodes 18 and/or location sensors 20. In this embodiment, elongated body 26 of guiding sheath 22 may be deflectable to impart control over the advancement of guiding sheath 22 through the vasculature of the patient and/or to help control which areas of the patient's anatomy are contacted by lasso electrode assembly 16. At least one puller wire 30 may be secured at its distal end to an anchor within elongated body 26 at a distal portion of guiding sheath 22 and at its proximal end to actuator 32 on control handle 24. Rotating, or otherwise manipulating actuator 32 may place puller wire 30 under tension, producing a deflection of elongated body 26 away from its longitudinal axis. One puller wire may be employed to impart a uni-directional deflection, while an additional puller wire may provide bi-directional deflection. Further details regarding deflectable guiding sheaths may be found in U.S. Patent No. 8,197,464, entitled "Deflecting Guide Catheter For Use In A Minimally Invasive Medical Procedure For The Treatment Of Mitral Valve Regurgitation". In other embodiments, electrophysiologic catheter 10 may also be deflectable. Examples of suitable construction details for deflectable catheters for are described in U.S. Patent No. 7,377, 906, entitled "Steering Mechanism For Bi-Directional Catheter," and U.S. Patent No. 8,137,308, entitled "Catheter With Adjustable Deflection Sensitivity". Other suitable techniques may also be employed to provide deflection as desired.

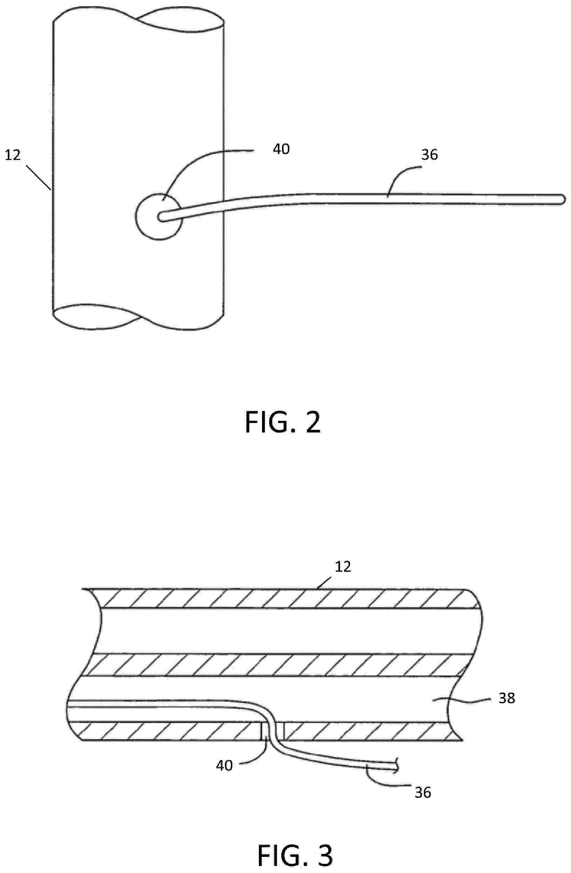

According to the techniques of this disclosure, the need to use a separate metallic component to form the ring electrode may be avoided. As schematically shown in FIGs. 2-5, ring electrodes 18 and/or location sensors 20 may be constructed from a helical coil 34 of a suitable length of lead wire 36. Lead wire 36 may be made of any suitable electrically conductive material, such as a non-oxidizing metal and may have any suitable diameter. In one embodiment, lead wire 36 may be 0.003 inch (approx. 0.076 mm) MONEL^{®} 400 wire, a high tensile strength nickel-copper alloy coated with a nonconductive coating. Notably, these techniques allow electrode 18 and/or location sensor 20, as well as the associated lead wire 36, to be formed from a single, continuous length of wire so that no separate electrical connection, such as a weld or solder joint, between the component and lead wire is necessary, facilitating manufacture and improving reliability.

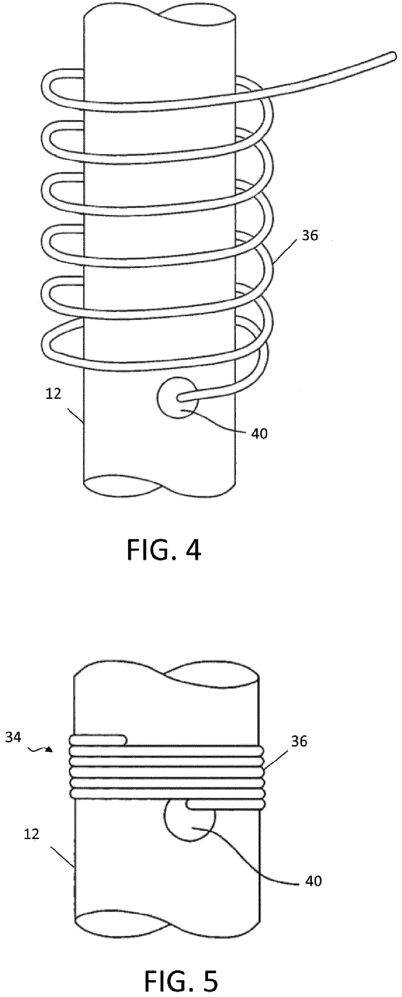

Initially, lead wire 36 may be routed through lumen 38 of elongated body 12 with respect to electrophysiologic catheter 10 as shown in FIG. 2 and in the corresponding cross sectional view of FIG. 3. These techniques may also be applied with respect to elongated body 26 of guiding sheath 22. As will be appreciated, lead wire 36 may extend proximally through the length of elongated body 12 to control handle 14, where a suitable coupling may be provided to create connection to the appropriate equipment to receive or transmit electrical signals. A distal length of lead wire 36 is pulled to extend from opening 40 in the side wall of elongated body 12 that communicates with lumen 38. The distal length of lead wire 36 may be of sufficient length to form a desired number of wraps around elongated body 12 as depicted in FIG. 4. Opening 40 may be formed, for example, by inserting a needle through the wall of elongated body 12 and heating the needle sufficiently to form a permanent hole. Opening 40 may be sufficiently large to enable lead wire 36 to be pulled through, such as by a microhook or the like, and yet sufficiently small to be easily sealed. In some embodiments, opening 40 may be filled with a suitable material, such as a polyurethane resin, to seal elongated body 12.

The number of wraps may be selected to form coil 34 with properties as warranted to function as an electrode 18 or as a location sensor 20 according to the description below. Notably, to form electrode 18, any insulation may be stripped from the distal portion of lead wire 36 so that adjacent turns are electrically coupled. Alternatively, the insulation may be left intact when forming location sensor 20, so that coil 34 may generate or respond to a magnetic field for use in an impedance-based positioning system as described below. As indicated in FIG. 5, the wraps of lead wire 36 may be tightened to remove any slack and to cause the turns to abut each other, thereby completing coil 34. As desired, an area of elongated body 12 may be heated to a temperature sufficient to soften the material, so that further tightening of lead wire 36 may help embed the wire in the side wall of catheter body 12.

As noted, the number of wraps used in coil 34 may be tailored to provide either an electrode 18 or a location sensor 20 having desired properties. For example, the number of wraps of lead wire 36 used to form coil 34 having sufficient surface area when functioning as electrode 18. In one embodiment, longitudinal length of coil 34 may be in the range of approximately 1 mm to 4 mm and it will be appreciated that the number of wraps depends at least in part on the diameter of lead wire 36. The desired characteristics of coil 34 may also be adjusted depending on whether the resulting electrode 18 is intended to function as a diagnostic electrode for measuring electrical signals, to function as an ablation electrode for delivering energy, or to function as both. Any suitable number of electrodes 18 may be formed by separate coils 34, and they may be distributed in a desired position along the length of elongated body 12. Further, electrodes 18 may be configured as electrode pairs to function as bipolar electrodes, such that they may be constructed from two coils 34. Therefore, by following the techniques of this disclosure, coil 34 may be formed as electrode 18 in a manner that permits opening 40 to be sealed and its integrity confirmed as compared to conventional methods for mounting ring electrodes, which require the lead wire to be drawn back into the lumen the ring electrode is position, preventing sealing and inspection of the opening. Further, since coil 34 is formed from separate wraps of lead wire 36, it may accommodate bends and other deformations in elongated body 12 more readily than a conventional monolithic ring electrode. Alternatively or in addition, one or more electrodes 18 may be formed from coils 34 around elongated body 26 of guiding sheath 22.

Similarly, a suitable number of wraps may be formed when coil 34 is intended to function as location sensor 20. As will be appreciated, coil 34 may be used with a system for generating three-dimensional position information regarding catheter. For example, coil 34 may be configured to generate electrical signals in response to an externally applied magnetic field, such as by responding to specific frequency of the field. Exemplary details regarding certain embodiments of location sensors 20 may be found in commonly-assigned U.S. Patent No. 8,792,296, entitled "Catheter With Single Axial Sensors". As with electrodes 18, one or more location sensors 20 formed from coils 34 may be positioned as desired on elongated body 12 of catheter 10, on elongated body 26 of guiding sheath 22, or both.

The elongated body 12 is flexible, i.e., bendable, but substantially non-compressible along its length. The elongated body 12 can be of any suitable construction and made of any suitable material. One construction comprises an outer wall made of polyurethane or PEBAX^{®} (polyether block amide). The outer wall comprises an imbedded braided mesh of stainless steel or the like to increase torsional stiffness of the elongated body 12 so that, when the control handle 14 is rotated, the distal end of the elongated body will rotate in a corresponding manner. In other embodiments, an inner stiffening tube may be coaxially disposed within a lumen of elongated body 12, forming an annular lumen 38 through which lead wires 36 for electrodes 18 and/or location sensors 20 may be routed. In some embodiments, elongated body 12 may be steerable and/or deflectable using any suitable technique, which are known to those of ordinary skill in the art. The outer diameter of the elongated body 12 is not critical, but generally should be as small as possible and may be no more than about 10 french depending on the desired application. For example, for use in the mapping and ablation for isolation of a pulmonary vein, elongated body may have an outer diameter of about 7 to 7.5 french. Likewise the thickness of the outer wall is not critical, but may be thin enough so that the central lumen can accommodate lead wires 24 and any other necessary components. If desired, the inner surface of the outer wall is lined with a stiffening tube (not shown) to provide improved torsional stability. An example of a elongated body construction suitable for use in connection with the present invention is described and depicted in U.S. Patent No. 6,064,905.

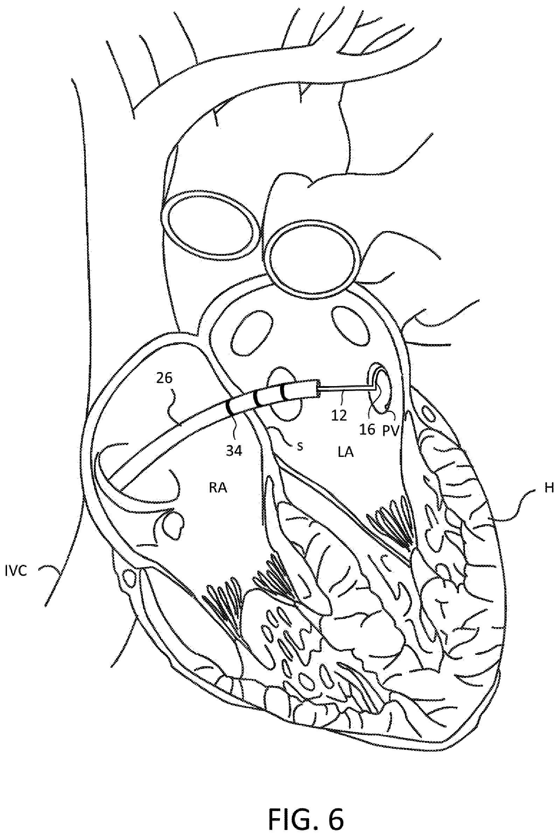

In one aspect, an electrophysiologist may introduce guiding sheath 22, a guidewire and a dilator into the patient, as is generally known in the art. As an example, guiding sheath 22 may be similar to a PREFACE^{™} Braided Guiding Sheath (commercially available from Biosense Webster, Inc., Diamond Bar, CA). The guidewire is inserted, the dilator is removed, and catheter 10 is introduced through the guiding sheath 22. In one exemplary procedure as depicted in FIG. 6, the catheter 10 is first introduced through guiding sheath 22 to the patient's heart (H) through the right atrium (RA) via the inferior vena cava (IVC), where it passes through the septum (S) in order to reach the left atrium (LA). Depending on how far catheter 10 is advanced through guiding sheath 22, electrodes and/or location sensors on elongated body 12 may be blocked by guiding sheath 22. Correspondingly, providing one or more electrodes and/or location sensors on elongated body 26 of guiding sheath 22 may permit their use regardless of the relative positioning of catheter 10. In one aspect, this may facilitate visualization of guiding sheath 22 within the patient before catheter has been fully advanced.

As will be appreciated, lasso electrode assembly 16 may be deflected into a straightened configuration and constrained within guiding sheath 22 to allow catheter 10 to be passed through the patient's vasculature to the desired location. Once the distal end of the catheter reaches the desired location, e.g., the left atrium, guiding sheath 22 is withdrawn to expose the lasso electrode assembly 16, where it recoils into its arcuate configuration. With the lasso electrode assembly 16 then positioned in the ostium of a pulmonary vein (PV), electrodes 18 contact the ostial tissue and may be used to map electrical signals in this area. In other embodiment, different electrode configuration may be used to access other areas of a patient's anatomy as desired.

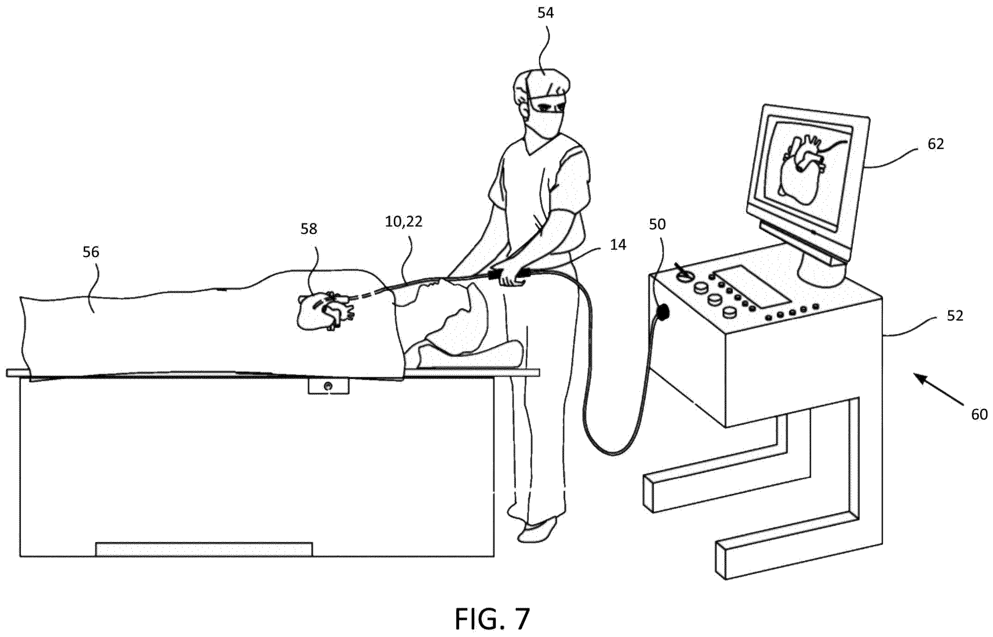

To help illustrate use of catheter 10 and guiding sheath 22, FIG. 7 is a schematic depiction of an invasive medical procedure, according to an embodiment of the present invention. Catheter 10, with the lasso electrode assembly 16 (not shown in this view) at the distal end and/or guiding sheath 22 may have a connector 50 at the proximal end for coupling the lead wires 36 and/or others from their respective electrodes 18 formed from coils 34 (not shown in this view) to a console 52 for recording and analyzing the signals they detect as well as for supplying ablating energy. An electrophysiologist 54 may insert the catheter 10 through guiding sheath 22 into a patient 56 in order to acquire electropotential signals from the heart 58 of the patient. The electrophysiologist 54 uses control handles 14 and 24 to perform the insertion. Console 52 may include a processing unit 60 which analyzes the received signals, and which may present results of the analysis on a display 62 attached to the console. The results are typically in the form of a map, numerical displays, and/or graphs derived from the signals. Processing unit 60 may also control the delivery of energy to one or more electrodes 18 for creating one or more lesions.

Further, the processing unit 60 may also receive signals from location sensors 20 (not shown in this view). As noted, the sensor(s) may each comprise a magnetic-field-responsive coil or a plurality of such coils, each formed by coil 34. Using a plurality of coils enables six-dimensional position and orientation coordinates to be determined. The sensors may therefore generate electrical position signals in response to the magnetic fields from external coils, thereby enabling processor 60 to determine the position, (e.g., the location and orientation) of the distal end of catheter 10 and/or guiding sheath 22 within the heart cavity. The electrophysiologist may then view the position of the lasso electrode assembly 16 on an image the patient's heart on the display 62. By way of example, this method of position sensing may be implemented using the CARTO^{™} system, produced by Biosense Webster Inc. (Diamond Bar, Calif.) and is described in detail in U.S. Pat. Nos. 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1. As will be appreciated, other location sensing techniques may also be employed. The coordinates of location sensor 20 may be determined and, with other known information pertaining to the configuration of lasso electrode assembly 16, used to find the positions of each of the electrodes 18.

The preceding description has been presented with reference to presently disclosed embodiments of the invention. Workers skilled in the art and technology to which this invention pertains will appreciate that alterations and changes in the described structure may be practiced without meaningfully departing from the principal, scope of this invention, defined in the appended claims. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims.

## Claims

1. A electrophysiologic device comprising:
an elongated body (12) having proximal and distal ends and at least one lumen (38) therethrough;
a first coil (34) configured as a location sensor (20) and wound around a side wall of a distal portion of the elongated body, wherein the first coil comprises a plurality of helical wraps of a first continuous wire (36) that extends to the proximal end of the elongated body through the at least one lumen, wherein the first wire is routed through a first opening (40) in the side wall of the distal portion of the elongated body, and wherein the first opening communicates with the at least one lumen; and
a second coil (34) configured as a ring electrode (18) and wound around the side wall of the distal portion of the elongated body, wherein the second coil comprises a plurality of helical wraps of a second continuous wire (36) that extends to the proximal end of the elongated body through the at least one lumen, wherein the second wire is routed through a second opening (40) in the side wall of the distal portion of the elongated body, and wherein the second opening communicates with the at least one lumen.

2. The electrophysiologic device of claim 1, wherein the electrophysiologic device comprises an electrophysiologic catheter.

3. The electrophysiologic device of claim 1, wherein the electrophysiologic device comprises a guiding sheath (22) having an inner lumen (28) configured to coaxially receive a catheter

4. The electrophysiologic device of claim 3, wherein the guiding sheath is deflectable.

5. The electrophysiologic device of claim 4, wherein the first or second coil has a longitudinal length in the range of approximately 1 mm to 4 mm.

6. The electrophysiologic device of claim 1, further comprising an additional coil configured as a ring electrode (18) or a location sensor.

7. A method for constructing an electrophysiologic device comprising:
providing an elongated body (12) having proximal and distal ends and at least one lumen (38) therethrough;
routing a first continuous wire (36) from the proximal end of the elongated body through the at least one lumen to a distal portion and forming a plurality of wraps around a side wall of the elongated body at the distal portion to form a first coil (34) configured to function as a location sensor (20), and routing the first wire through a first opening (40) in the side wall of the elongated body at the distal portion, and wherein the first opening communicates with the at least one lumen; and
routing a second continuous wire (36) from the proximal end of the elongated body through the at least one lumen to the distal portion and forming a plurality of wraps around the side wall of the elongated body at the distal portion to form a second coil (34) configured to function as a ring electrode (18), and routing the second wire through a second opening (40) in the side wall of the elongated body at the distal portion, and wherein the second opening communicates with the at least one lumen.

8. The method of claim 7, further comprising removing insulation from a distal portion of the second wire that extends from the opening.

## Patentansprüche

1. Elektrophysiologische Vorrichtung, umfassend:
einen länglichen Körper (12), der ein proximales und ein distales Ende und mindestens ein Lumen (38) dahindurch aufweist;
eine erste Spule (34), die als ein Positionssensor (20) konfiguriert und um eine Seitenwand eines distalen Abschnitts des länglichen Körpers gewickelt ist, wobei die erste Spule eine Vielzahl von spiralförmigen Wicklungen eines ersten durchgehenden Drahts (36) umfasst, der sich durch das mindestens eine Lumen zu dem proximalen Ende des länglichen Körpers erstreckt, wobei der erste Draht durch eine erste Öffnung (40) in der Seitenwand des distalen Abschnitts des länglichen Körpers geführt ist und wobei die erste Öffnung mit dem mindestens einen Lumen in Verbindung steht; und
eine zweite Spule (34), die als eine Ringelektrode (18) konfiguriert und um die Seitenwand des distalen Abschnitts des länglichen Körpers gewickelt ist, wobei die zweite Spule eine Vielzahl von spiralförmigen Wicklungen eines zweiten durchgehenden Drahts (36) umfasst, der sich durch das mindestens eine Lumen zu dem proximalen Ende des länglichen Körpers erstreckt, wobei der zweite Draht durch eine zweite Öffnung (40) in der Seitenwand des distalen Abschnitts des länglichen Körpers geführt ist und wobei die zweite Öffnung mit dem mindestens einen Lumen in Verbindung steht.

2. Elektrophysiologische Vorrichtung nach Anspruch 1, wobei die elektrophysiologische Vorrichtung einen elektrophysiologischen Katheter umfasst.

3. Elektrophysiologische Vorrichtung nach Anspruch 1, wobei die elektrophysiologische Vorrichtung eine Führungshülle (22) umfasst, die ein inneres Lumen (28) aufweist, das konfiguriert ist, um einen Katheter koaxial aufzunehmen.

4. Elektrophysiologische Vorrichtung nach Anspruch 3, wobei die Führungshülle ablenkbar ist.

5. Elektrophysiologische Vorrichtung nach Anspruch 4, wobei die erste oder die zweite Spule eine Längslänge in dem Bereich von etwa 1 mm bis 4 mm aufweist.

6. Elektrophysiologische Vorrichtung nach Anspruch 1, ferner umfassend eine zusätzliche Spule, die als eine Ringelektrode (18) oder ein Positionssensor konfiguriert ist.

7. Verfahren zum Konstruieren einer elektrophysiologischen Vorrichtung, umfassend:
Bereitstellen eines länglichen Körpers (12), der ein proximales und ein distales Ende und mindestens ein Lumen (38) dahindurch aufweist;
Führen eines ersten durchgehenden Drahts (36) von dem proximalen Ende des länglichen Körpers durch das mindestens eine Lumen zu einem distalen Abschnitt und Ausbilden einer Vielzahl von Wicklungen um eine Seitenwand des länglichen Körpers an dem distalen Abschnitt, um eine erste Spule (34) auszubilden, die konfiguriert ist, um als ein Positionssensor (20) zu arbeiten, und Führen des ersten Drahts durch eine erste Öffnung (40) in der Seitenwand des länglichen Körpers an dem distalen Abschnitt, und wobei die erste Öffnung mit dem mindestens einen Lumen in Verbindung steht; und
Führen eines zweiten durchgehenden Drahts (36) von dem proximalen Ende des länglichen Körpers durch das mindestens eine Lumen zu dem distalen Abschnitt und Ausbilden einer Vielzahl von Wicklungen um die Seitenwand des länglichen Körpers an dem distalen Abschnitt, um eine zweite Spule (34) auszubilden, die konfiguriert ist, um als eine Ringelektrode (18) zu arbeiten, und Führen des zweiten Drahts durch eine zweite Öffnung (40) in der Seitenwand des länglichen Körpers an dem distalen Abschnitt, und wobei die zweite Öffnung mit dem mindestens einen Lumen in Verbindung steht.

8. Verfahren nach Anspruch 7, ferner umfassend ein Entfernen einer Isolierung von einem distalen Abschnitt des zweiten Drahts, der sich von der Öffnung erstreckt.

## Revendications

1. Dispositif électrophysiologique comprenant :
un corps allongé (12) ayant des extrémités proximale et distale et au moins une lumière (38) traversant celui-ci ;
une première bobine (34) configurée comme un capteur d'emplacement (20) et enroulée autour d'une paroi latérale d'une partie distale du corps allongé, dans lequel la première bobine comprend une pluralité d'enroulements hélicoïdaux d'un premier fil continu (36) qui s'étend jusqu'à l'extrémité proximale du corps allongé à travers l'au moins une lumière, dans lequel le premier fil est acheminé à travers une première ouverture (40) dans la paroi latérale de la partie distale du corps allongé, et dans lequel la première ouverture communique avec l'au moins une lumière ; et
une seconde bobine (34) configurée comme une électrode annulaire (18) et enroulée autour de la paroi latérale de la partie distale du corps allongé, dans lequel la seconde bobine comprend une pluralité d'enroulements hélicoïdaux d'un second fil continu (36) qui s'étend jusqu'à l'extrémité proximale du corps allongé à travers l'au moins une lumière, dans lequel le second fil est acheminé à travers une seconde ouverture (40) dans la paroi latérale de la partie distale du corps allongé, et dans lequel la seconde ouverture communique avec l'au moins une lumière.

2. Dispositif électrophysiologique selon la revendication 1, dans lequel le dispositif électrophysiologique comprend un cathéter électrophysiologique.

3. Dispositif électrophysiologique selon la revendication 1, dans lequel le dispositif électrophysiologique comprend une gaine de guidage (22) ayant une lumière interne (28) conçue pour recevoir coaxialement un cathéter

4. Dispositif électrophysiologique selon la revendication 3, dans lequel la gaine de guidage est déformable.

5. Dispositif électrophysiologique selon la revendication 4, dans lequel la première ou la seconde bobine a une longueur longitudinale comprise entre environ 1 mm et 4 mm.

6. Dispositif électrophysiologique selon la revendication 1 comprenant en outre une bobine supplémentaire configurée comme une électrode annulaire (18) ou un capteur d'emplacement.

7. Procédé de construction d'un dispositif électrophysiologique comprenant :
la fourniture d'un corps allongé (12) ayant des extrémités proximale et distale et au moins une lumière (38) à travers celui-ci ;
l'acheminement d'un premier fil continu (36) de l'extrémité proximale du corps allongé à travers l'au moins une lumière jusqu'à une partie distale et la formation d'une pluralité d'enroulements autour d'une paroi latérale du corps allongé au niveau de la partie distale pour former une première bobine (34) configurée pour fonctionner comme un capteur d'emplacement (20), et l'acheminement du premier fil à travers une première ouverture (40) dans la paroi latérale du corps allongé au niveau de la partie distale, et dans lequel la première ouverture communique avec l'au moins une lumière ; et
l'acheminement d'un second fil continu (36) de l'extrémité proximale du corps allongé à travers l'au moins une lumière jusqu'à la partie distale et la formation d'une pluralité d'enroulements autour de la paroi latérale du corps allongé au niveau de la partie distale pour former une seconde bobine (34) configurée pour fonctionner comme une électrode annulaire (18), et l'acheminement du second fil à travers une seconde ouverture (40) dans la paroi latérale du corps allongé au niveau de la partie distale, et dans lequel la seconde ouverture communique avec l'au moins une lumière.

8. Procédé selon la revendication 7, comprenant en outre le retrait de l'isolation d'une partie distale du second fil qui s'étend à partir de l'ouverture.
